# EUROPEAN PATENT SPECIFICATION

(11) **EP 1 455 805 B1**
(45) Date of publication and mention of the grant of the patent: **16.05.2007**
(21) Application number: 99952039.8
(22) Date of filing: 26.10.1999
(51) Int. Cl.: A61K 33/30, A61K 9/06, A61K 47/02, A61K 47/06, A61P 17/00, A61K 33/22

(54) **A PROCESS FOR OBTAINING A ZINC OXIDE CONTAINING PRODUCT FOR SKIN-HEALING, CARE AND PROTECTION IN CHILDREN AND ADULTS**
VERFAHREN ZUR HERSTELLUNG EINES ZINKOXID ENTHALTENDEN PRODUKTES ZUR HEILUNG, PFLEGE UND ZUM SCHUTZ DER HAUTVON ERWACHSENEN UND KINDERN
PROCEDE RELATIF A L'OBTENTION D'UN PRODUIT A BASE D'OXYDE DE ZINC POUR TRAITEMENT, ENTRETIEN ET PROTECTION DE LA PEAU CHEZ L'ENFANT ET L'ADULTE

(43) Date of publication of application: 15.09.2004
(73) Proprietor: Pavlovic, Vojislav, 11118 Beograd 32 (YU)
(72) Inventor: Pavlovic, Vojislav, 11118 Beograd 32 (YU)
(74) Representative: Matschnig, Franz
(86) International application number: PCT/YU1999/000009
(87) International publication number: WO 2001/030363

(56) References cited:
- US-A- 4 143 159
- US-A- 4 382 765
- US-A- 5 951 993

## Description

A procedure for obtaining a product for skin-healing, care and protection in children and adults belongs to a field of pharmacology, namely to the products for medical or cosmetic use, more precisely to a field of medicaments, i. e. medical products characterized by a special physical form- to salves.

The invention solves the technical problem which consists in how to define both, a ratio between lanolin, vaseline, almond water and other components and the procedure for obtaining a product for healing various skin diseases, which could be used for skin-care and protection but not diminishing the medical effect of the basic components.

The similar products with specific pharmaco-dynamic characteristics for healing skin deases are known, however, it is known as well that the indication range for use of such products is often very narrow, that they are anticipated for external use and some of them may have a cumulative effect and cause allergic manifestations after a long-term use.

By quoting some of the characteristics of the known products, we indicated at the same time their bad sides, which have been rectified by the product with a content pursuant to this invention and made after the original procedure, thus the advantage of this product over the known products has been enhanced, firstly in the size of the indication field and due to the use of the absolutely nontoxic components, there are no contraindications, no cumulative effect and no allergic reactions.

The essence of the invention lies in the fact that the product with desired pharmaco-dynamic characteristics is obtained from zync oxyde powder, talcum powder, boric acid powder, distille water, almond water, paraffin oil, lanolin and vaselin. It will have a curative, protective and preventive (in cosmetics) effects and will enhance vascularization (urging the healing up of wounds), having at the same time a mild keratolytic and nutritious effect (elimination of cellulite scars). The product obtained by this original precedure is used effectively in children and adults.

Regarding the aforementioned, the indication field of this product is wide:
- rash (intertriggo);
- crusts (crustae);
- bums (combustio);
- phase of blistered changes (blisters not being opened);
- epithelization phase;
- dry face;
- dry skin;
- wounds;
- cellulite scars (striae).

According to the invention, the procedure for obtaining a product for skin-healing, care and protection in children and adults commences with weighing the initial components on a precise (apothecary) scales.

Then the zync oxyde (Pulvis Zynzi Oxydarti) in quantity of 0,47 - 0,70 % and talcum powder (Pulvis Talci veneti) in quantity of 0,47 - 0,70 % are dissolve in 1 % of paraffin oil (Oleum Parafini in duration of 1 - 2 minutes, so that the components are homogenized and prepared for blending with other components.

Lanolin (Lanolini), in quantity of 27,06 - 31.76 %, vaselin (Vaselini) in quantity of 27,06 -31.76 %, paraffin (Oleum Parafini) in quantity of 8,41 - 13,12 %, distille water (Aquae destillatae), in quantity of 11,76 %, boric acid powder (Pulvis Acidi Borici) in quantity of 4,70 % and almond water (Aquae Laniocerassi (Amygdallae)) in quantity of 9,41 - 14,12 % are put into in a paten and melted in a water bath until completely melted at the temperature of approx. 65°C.

When the components are melted, they are removed from the heat and a homogenized mixture consisting from zync oxyde, talcum powder and paraffin oil is added slowly, with constant stirring until the compound is completely homogenized, i. e. until the consistency of grease is reached.

Depending on the quantity, a pastille or a mixer is used respectively for the preparation.

### EXAMPLE

Zync oxyde powder in quantity of 0,4 gr and talcum powder in quantity of 0,6 gr are dissolve in 1 gr of paraffin oil in duration of 1-2 minutes until completely homogenized.

Lanolin in quantity of 27 gr, vaselin in quantity of 23 gr, paraffin oil in quantity of 11 gr, distille water in quantity of 10 gr, boric acid powder in quantity of 4 gr and almond water in quantity of 8 gr are put in a paten where they are melted in a water bath at the temperature of approx. 65°C until completely melted.

When the components are melted, the compound is removed from the heat and a homogenized mixture consisting from zync oxyde, talcum powder and paraffin oil is slowly added, with a constant stirring until completely homogenized.

When the compound is completely cooled down, a mild yellowish ointment with an almond scent, in the quantity of 85 gr, is the product obtained by this procedure.

Depending on the quantity, a pastille or a mixture is used respectively for the preparation.

The product is a little bit more greasy and easily spreadable.

The product (ointment) prepared in this way is kept on a dry, cool and dark place (in a refrigerator at + 4° C).

Beside this basic version, it is possible to define new variants by putting the emphasis on the special pharmacological characteristics.

Depending on use, the product is packed in corresponding package (plastic containers, glass jars, tubes...)

## Claims

1. A process for obtaining a product for skin-healing, care and protection, ***characterized in that*** zync oxyde powder in quantity of 0,47 - 0,70 % and talcum powder in quantity of 0,47 - 0,70 .% are dissolved in 1 % of paraffin oil in duration of 1-2 minutes until completely homogenized, that lanolin in quantity of 27,06 - 31,76 %, vaselin in quantity of 27,06 - 31,76 %, paraffin oil in quantity of 8,41 - 13,12 %, distilled water in quantity of about 11,76 %, boric acid powder in quantity of about 4,70 % and almond water in quantity of 9,41 - 14,12 % are melted at the temperature of approx. 65°C until the compound is completely melted and homogenized; the compound is removed from the heat and the homogenized mixture consisting from zync oxyde, talcum powder and paraffin oil is added slowly, with constant stirring until completely homogenized.

## Patentansprüche

1. Verfahren zur Herstellung eines Produkts zur Hautheilung, -pflege und -schutz, **dadurch gekennzeichnet, dass**
Zinkoxidpulver in einer Menge von 0,47 bis 0,70% und Talkumpulver in einer Menge von 0,47 bis 0,70% in 1% Paraffinöl für eine Dauer von 1 bis 2 Minuten bis zur völligen Homogenisierung gelöst wird,
Lanolin in der Menge von 27,06 bis 31,76%, Vaseline in der Menge von 27,06 bis 31,76%, Paraffinöl in der Menge von 8,41 bis 13,12%, destilliertes Wasser in der Menge von etwa 11,76%, Borsäurepulver in der Menge von etwa 4,70% und Mandelwasser in der Menge von 9,41 bis 14,12% bei einer Temperatur von etwa 65°C geschmolzen werden, bis das Gemisch vollständig geschmolzen und homogenisiert ist;
das Gemisch von der Hitze entfernt wird und
die homogene Mischung, bestehend aus Zinkoxid, Talkumpulver und Paraffinöl langsam, unter konstantem Rühren bis zu völligen Homogenisierung hinzugefügt wird.

## Revendications

1. Procédé d'obtention d'un produit de cicatrisation de la peau, de soins et de protection, **caractérisé en ce que** de la poudre d'oxyde de zinc en une quantité de 0,47 à 0,70 % et de la poudre de talc en une quantité de 0,47 à 0,70 % sont dissoutes dans 1 % d'huile de paraffine pendant une durée de 1 à 2 minutes jusqu'à ce qu'elles soient complètement homogénéisées, que de la lanoline en une quantité de 27,06 à 31,76 %, de la vaseline en une quantité de 27,06 à 31,76 %, de l'huile de paraffine en une quantité de 8,41 à 13,12 %, de l'eau distillée en une quantité d'environ 11,76 %, de la poudre d'acide borique en une quantité d'environ 4,70 % et de l'huile d'amande en une quantité de 9,41 à 14,12 % sont fondues à la température d'approximativement 65 °C jusqu'à ce que le composé soit complètement fondu et homogénéisé ; le composé est retiré de la chaleur et le mélange homogénéisé consistant en l'oxyde de zinc, la poudre de talc et l'huile de paraffine est ajouté lentement, avec agitation constante jusqu'à ce qu'il soit complètement homogénéisé.
